# EUROPEAN PATENT SPECIFICATION

(11) **EP 0 659 089 B1**
(45) Date of publication and mention of the grant of the patent: **24.11.1999**
(21) Application number: 93920379.0
(22) Date of filing: 26.08.1993
(51) Int. Cl.: A61L 15/44, A61L 15/42, A61L 15/28, A61L 15/62

(54) **LYOPHILIZED FOAM WOUND DRESSING**
GEFRIERGETROKNETER SCHAUMWUNDVERBAND
PANSEMENT POUR BLESSURE EN MOUSSE LYOPHILISEE

(30) Priority: 10.09.1992 US 942869
(43) Date of publication of application: 28.06.1995
(73) Proprietor: McNEIL-PPC, INC., Milltown New Jersey 08850 (US)
(72) Inventor: BRITTON, Peter, Scotch Plains, NJ 07076 (GB); COHEN, Jonathan, Marc, Pomona, NY 10970 (US); FINKENAUR, Amy, L., Neshanic Station, NJ 08853 (US); FLANAGAN, Patricia, Naperville, IL 60540 (US); SHALABY, Shalaby, W., Anderson, SC 29625 (US)
(74) Representative: Mercer, Christopher Paul
(86) International application number: US9308074
(87) International publication number: WO9405341

(56) References cited:
- EP-A- 0 063 604
- EP-A- 0 209 726
- EP-A- 0 238 839
- WO-A-91/19480
- GB-A- 1 378 931

## Description

### Background of the Invention

The invention relates to dressings that are useful for administering active ingredients to the wound. More particularly, it relates to wound dressings having water-soluble wound facing layers comprising lyophilized foams; methods of using such dressings for the controlled or sustained delivery of active ingredients to wounds; and methods for their manufacture. The wound dressings of the present invention can provide for the controlled and sustained release of an active ingredient for at least about 24 hours.

Chronic cutaneous wounds and ulcers represent a significant portion of hospital admissions, and the healing of these wounds is typically a costly and time-consuming endeavor that can take weeks and even months. Dressings that provide for the controlled or sustained release of active ingredients to accelerate healing are an integral part of the treatment regime.

Generally, four types of dressings are used to treat chronic wounds; namely, films, gels, hydrocolloids and foams. Dressings comprising films and gels are typically used to treat moderately exudative wounds, whereas hydrocolloid and foam-containing dressings are used to treat heavily exudative wounds. Ideally, such dressings should reduce the amount of care required to manage the injury and the cost of the care. In addition, the treatment regime should minimize the patient's pain and suffering.

There are several disadvantages associated with the present use of foams in wound dressings. Such foams typically dissolve too quickly and do not possess the requisite tactile and structural properties.

For example, in U.S. Patent No. 4,292,972, Pawelchak et al. disclose a lyophilized foam sponge product containing sodium carboxymethylcellulose, pectin, gelatin and a pharmaceutical. The colloidal dispersions described by Pawelchak do not aerate well, and the resulting foams are stiff, brittle and possess poor structural integrity. In addition, Pawelchak's foams dissolve in less than about five hours. Another disadvantage is that it is not possible to control the dosage delivery rates and dissolution times of foams using Pawelchak's method of manufacture.

In U.S. Patent No. 4,642,903, Davies discloses the use of freeze-dried foams for dispensing a variety of active ingredients. Davies' foams are designed to dissolve virtually instantaneously. As with Pawelchak, it is not possible to control the various properties of Davies' freeze-dried foams using Davies' process.

Japanese Kokai Patent Application No. 80-71532 describes a method for manufacturing polyvinyl-alcohol-based sponge materials that further contain various cellulose ether derivatives. These foams are designed to break down quickly in warm water; thus, like the foams previously described, they tend to dissolve very rapidly. Upon dissolution, the foams readily liquify and fail to form a viscous gel. The formation of such viscous gels is particularly important to the healing process because they offer a moist environment and good padding protection to the wound. Moreover, the method utilized to manufacture the foams suffers from the same disadvantages discussed above.

Japanese Kokai Patent Application No. 40-9431 describes a method for manufacturing sponges for hemostasis wherein the sponges are made from freeze-dried foams containing methylcellulose and a foam promoting agent such as gelatin or sodium lauryl sulfate.

Accordingly, a need exists for a wound dressing having a wound facing layer that provides for the sustained and/or controlled release of an active ingredient to a wound for at least several hours. Such a dressing should maintain a moist and protective healing environment, and yet be highly absorbent and minimize wound fluid strikethrough. The dressing should be soft, flexible, and should not reinjure the wound upon removal.

Furthermore, there is a need for a method of producing such wound dressings whereby various properties of the wound facing layers, including dissolution times and active ingredient delivery rates, can be substantially controlled and readily reproduced.

### Brief Description of the Drawings

Figure 1 depicts the release kinetics of EGF from lyophilized foams of the invention containing hydroxypropylmethylcellulose.
Figure 2 shows a partial thickness wound model using the wound dressings of the invention.

### Summary of the Invention

The inventors have discovered novel processes for manufacturing wound dressings having wound facing layers comprised of water-soluble lyophilized foams and active ingredients, which overcome the problems associated with prior art methods of manufacture and produces foams that are ideal for use in wound dressings.

The inventors have found that by controlling the liquid density of the foamed dispersion prior to lyophilization, it is possible to achieve excellent control of the properties of the freeze-dried foams including absorption capacity, flexibility, active ingredient release rate, dissolution time, dosage, and the like. It has further been determined that it is possible to control the liquid density of foamed dispersions by using a continuous enclosed mixer known as a Oakes foamer. The use of such a foamer makes it possible to manufacture foams (and wound dressings) having consistently reproducible properties. The use of an Oakes foamer for this purpose is heretofore unknown.

In a preferred embodiment, an aqueous dispersion comprising at least one water-soluble polymer and an active ingredient, preferably hydroxypropylmethylcellulose and a pharmaceutical, is prepared. The dispersion is then foamed using an Oakes foamer or the like. The dispersion aerates readily to form a sturdy liquid foam having active ingredient uniformly dispersed throughout the foam. Then the liquid foam dispersion is lyophilized, preferably at full-vacuum. The product can be made in the form of a sheet or cast into discrete shapes. The lyophilized foam may then be utilized as the wound facing layer of the absorbent pad of a wound dressing. Alternatively, the foam may make up the entire absorbent pad of the wound dressing. As used herein, wound facing layer refers to any portion of the wound dressing that comprises a soluble lyophilized foam and at least one active ingredient, that has a density of about 0.01 to about 0.1 gm/(cc)ml and a dissolution time of at least about 2 hours.

Dressings having wound facing layers made by the methods of the invention provide an excellent healing environment and alleviate many of the problems recited heretofore. Upon application to the wound, the wound facing layers of the invention readily absorb substantial amounts of wound exudate. As they absorb fluid, they slowly release the active ingredient to the wound. It has been found that wound facing layers of the invention can provide for the sustained and/or controlled release of an active ingredient for at least 2 hours. Preferably, the wound facing layers of the invention have dissolution times of at least about 4 hours to about 24 hours, thereby providing active ingredient to the wound for at least these periods.

The lyophilized foams of the wound dressings are soft, flexible and highly absorbent. They have an absorbent capacity of up to about 900% (by weight of the layer, in 1% saline solution) and can be used to treat even the most heavily exudative wounds.

Upon dissolution, the foam-containing dressings form viscous gels having excellent structural integrity and good cohesive strength. Consequently, the dressings provide good protection to the wound while it heals and prevent irritation or reinjury to the wound upon removal.

The unique combination of suitable materials, unique foaming processes and lyophilization provides a dressing that reduces the amount of care required to manage the injury. When placed under occlusion, they provide an ideal environment for the rapid healing of even chronic wounds.

Further objects and advantages of the present invention will be made known in the following description of the preferred embodiments and claims.

### Detailed Description of the Invention

The present invention provides a novel method for the manufacture of wound dressings having wound facing layers comprised of water-soluble lyophilized foams comprising at least one water soluble polymer and active ingredients. By using an enclosed mixer capable of operating under pressure to foam a polymer-containing dispersion and known lyophilization techniques, it is possible to produce lyophilized foams which are ideal for the treatment of virtually all types of wounds.

The wound facing layers of this invention are capable of absorbing many times its own weight of whole blood or body exudates. The products are also completely soluble. Thus, they can be employed to control bleeding in, for example, surgical procedures. The products can also be employed in the treatment of various open wounds such as burns, decubitus ulcers, venous stasis ulcers, diabetic ulcers, cutaneous ulcers, varicose ulcers, donor sites, psoriasis, skin incisions or any other accidental or medical injury that causes cell damage.

In another embodiment of the invention, the wound facing layers possesses wet-tack. Thus, these products can be employed as tissue adhesives for surgical procedures involving non-suturable tissue, in burn treatment, or as an adhesive in skin grafting procedures.

The wound dressings of this invention have water-soluble wound facing layers which provide for the sustained and/or controlled release of an active ingredient to the surface of a wound for a prolonged period. The term "sustained release" as used herein indicates that the concentration of the active ingredient is maintained at a relatively constant level on the surface of the wound. The term "controlled release" means that the active ingredient is delivered continuously over a period of time, say, for at least about two hours, preferably for at least about four hours, more preferably for at least about 8 hours, even more preferably for at least about 20 hours.

Besides the novel manufacturing process described herein, the physical characteristics of the foam-active ingredient containing wound facing layer are affected by several additional factors. These factors include the identity and molecular weight of the particular water-soluble polymers and active ingredient selected, and their respective concentrations in solution prior to foaming.

The first step in producing the wound facing layers of the invention comprises preparing an aqueous dispersion comprising at least one, and preferably several, water-soluble polymers, and an active ingredient. The term "aqueous dispersion" as used herein is meant to include dispersions (including solutions) in which the solvent is water and optionally, water-miscible liquids.

Preferably, the polymer is initially added to the liquid and dispersed, followed by addition and dispersion of the active ingredient. If necessary, heat can be applied to the mixture to facilitate dispersion.

Typically, polymer is added to the dispersion at a concentration of about 0.5% to 10% (by weight of the total dispersion including active ingredient), preferably about 1% to about 5%, even more preferably about 2% to about 3%. At lower concentrations, there may be insufficient polymer to prepare a sturdy foam, whereas at higher concentrations, the dispersion may be too viscous to foam under normal conditions.

Cellulose, cellulose ethers and derivatives thereof, and polymers of the type disclosed in U.S. patent No. 4,615,697, issued to Robinson, and commercially available under the generic name "polycarbophil" are suitable for use in the present invention.

Other suitable polymers include polyvinylpyrrolidone, polycarboxylated vinyl polymers, including polyacrylic acid polymers, polyacrylic acid polymers that are lightly crosslinked with a polyalkenyl polyether (such as those commercially available from B. Goodrich, Cincinnati, Ohio, under the trademarks, Carbopol® 434, 934P, 940 and 941), polysaccharide gums (such as natural plant exudates including e.g., karaya gum, ghatti gum and the like), and seed gums (including e.g., guar gum, locust bean gum, psigllium seed gum and the like). Cross-linked alginate gum gels of the type described in U.S. patent No. 3,640,741, to Etes are also suitable.

Preferably, the polymer is selected from the group consisting of polyurethanes, gelatins, celluloses and cellulose ethers, including hydroxypropylmethylcellulose, sodium carboxylmethylcellulose, methylcellulose, hydroxyethylcellulose, hydroxypropylcellulose, hydroxyethylmethylcellulose, hydroxyethylethylcellulose, hydroxypropylethylcellulose, carbopol, polyvinyl alcohol and derivatives thereof, dextran, chitosan and its derivatives, including chitosonium salts, starch and its derivatives, polyacrylamides, polyacrylates, agar, collagen, fibronectin, alginic acid and its salts, pectin, hyaluronic acid, or mixtures thereof.

Foams comprised of cellulose ethers are especially preferred. In particular, it has been found that foams comprising hydroxypropylmethylcellulose (HPMC), sodium carboxymethylcellulose (CMC), methylcellulose, gelatin or mixtures thereof, possess excellent qualities. Hydroxypropylmethylcellulose is most preferred.

Certain polymers, such as cellulose ethers generally and hydroxypropylmethylcellulose in particular, may be employed to provide liquid foams having good stability and structural integrity, and dry foams with desirable softness. Other polymers, like gelatin, may be incorporated in the wound facing layers of the invention to make them more rigid. One skilled in the art can readily determine the polymeric ingredients and their amounts that result in a foam having the preferred combination of suitable properties.

Various active ingredients may be incorporated into the devices, including for example, pharmaceuticals, nutrients and diagnostics, but active ingredients comprising pharmaceuticals are preferred. The active ingredient may be selected from the group of analgesics, anorexics, anti-arthritics, anti-bacterials, antibiotics, anti-convulsants, anti-depressants, anti-diabetics, anti-fungal agents, antihistamines, anti-hypertensives, anti-inflammatories, anti-microbials, anti-neoplastics, anesthetics, anti-virals, cardiovascular, vasodilators, vasoconstrictors, decongestants, diuretics, hormones, muscle relaxants, serotonin antagonists, tranquilizers, polypeptides, vitamins and the like.

Growth factors are especially preferred. Such preferred growth factors include EGF, acidic and basic fibroblast growth factors, α and β tumor derived growth factors, platelet derived growth factors and insulin-like growth factors.

The required application dosage and the density of the liquid foam to be generated dictate the required concentration of active ingredient to be added to the liquid dispersion. The active ingredient may be provided in the dispersion at a concentration of about 0.0001% to about 25% (by weight of the total dispersion), with about 0.001% to about 10% being preferred. The active ingredient may be present at from about 1% to about 95% by weight of the (dry) device, with about 5% to about 75% by weight being preferred, and about 5% to 50% being more preferred.

The amount of active ingredient incorporated into the wound facing layer of the dressing affects its therapeutic efficacy, its tactile and structural properties, and the ease with which it may be manufactured. For example, foams containing relatively higher percentages of active ingredient may result in an increase in the level of active ingredient available for absorption through the wound and surrounding skin layer. However, such foams may have inadequate dissolution times and an unpleasant feel.

Foams having lower percentages of active ingredient may be too brittle. Accordingly, it is necessary to empirically determine the amount of active ingredient that results in a wound dressing having the preferred combination of suitable properties.

The wound facing layers of the invention may further contain additional materials including, but not limited to, preservatives, fillers, excipients, binders, plasticizers, surfactants, wetting agents or penetration agents. Any such materials should be pharmaceutically acceptable and compatible with the other constituents of the foam. In addition, such ingredients may be added to tailor the performance of the product. For example, mannitol can be added to increase the rate of water penetration.

All materials incorporated into the foams should be blended into a homogeneous mixture (in the aqueous dispersion) prior to foaming. It is another advantage of the present invention that even insoluble materials like ketanserin may be added to the foams so long as all such materials are uniformly distributed within the foamed dispersion prior to freeze drying.

In order to ensure that the aqueous dispersion will subsequently foam, the viscosity of the dispersion should be maintained at about 4500 to 7000 cps, preferably about 5000 to 6000 cps, most preferably about 5600 cps, as measured in a Brookfield viscometer at 32°C using a number 4 spindle at 20 rpm. Accordingly, it may be necessary to cool the dispersion, preferably to about 32 to 35 °C, with mixing in order to maintain its viscosity.

After all materials to be incorporated into the foam have been blended into an aqueous dispersion having adequate viscosity for foaming, the dispersion is transferred to a continuous, enclosed mixer known as an "Oakes" foamer or the like. An Oakes foamer is capable of operating under pressure to foam the dispersion, and typically is used to manufacture creamy, smooth food products like ice cream and marshmallows. U.S. patent Nos. 2,572,049, 2,600,569, 2,679,866, and 3,081,069 describe various foamers useful for practicing the method of the invention, and the disclosures of these patents are incorporated herein by reference. The model referred to as the Oakes 2" Mixer, Model No. # 2MT.5A is especially preferred for use in practicing the invention.

A suitable foamer like the Oakes foamer is comprised of an electrical system, an air system and a product section. Generally, it comprises a pump; a mixing chamber; a head assembly having a rotor; a gas inlet; an outlet for the foamed dispersion; means to measure pump speed, rotor speed, flow rate, and pressure of an incoming gas; and means to measure the back pressure of the foamed dispersion.

The electrical system consists of a main power switch, and two independently variable speed controllers and motors with digital tachometers to measure the rotor and pump speed.

The air system consists of a manual on/off toggle valve, pressure regulator and gauge, an adjustable flow valve and meter and a one way (check) valve.

The product section consists of a positive displacement pump; speed reducer; inlet piping; a back pressure gauge to monitor back pressure and a mixing chamber. The gauge is isolated from the product by a diaphragm seal assembly. A liquid dispersion is fed to the pump, transmitted through a line to the mixing chamber wherein it is combined with air under pressure, and mixed by the head assembly with rotor. In the mixing chamber, the dispersion is foamed and the air and the dispersion are blended into a substantially uniform, homogeneous mixture. From the mixing chamber, the foamed dispersion is then sent to an outlet pipe.

It has been determined that the operating parameters of the mixer are primarily accountable for the density of the liquid foam, and consequently, for the properties of the foam produced. For example, increasing the pressure and/or flow rate of air into a fixed volume of dispersion generally produces a more flexible, faster dissolving foam. Similarly, changing the pump speed and/or the rotor speed, also changes the liquid density of the foamed liquid dispersion. The use of an enclosed foamer like an Oakes foamer permits each of these process variables to be separately monitored and independently altered in a controlled manner. Thus, it is possible to empirically determine the settings of the foamer which will produce a foamed dispersion having a desirable liquid density and, upon subsequent lyophilization, foams (and wound facing layers) having suitable qualities. Moreover, the use of such a foamer makes it possible to accurately reproduce the settings so that batches of foams having substantially identical properties and dosages are readily manufactured in each manufacturing sequence.

As indicated above, it has been found that the foam-containing portions of the wound dressing should have a (dry) density of about 0.001 to about 0.1 gm/(cc)ml, more preferably about 0.01 to about 0.05 gm/(cc)ml, and even more preferably about 0.01 to about 0.03 gm/(cc)ml, as determined using techniques which are well-known to those of ordinary skill in the art. Wound facing layers having densities within these ranges possess adequate dissolution times. In addition, such wound facing layers are sufficiently sturdy, yet soft and flexible, so that the dressings are comfortable to the user.

Typically, in order to achieve foams with desirable physical characteristics, adequate dissolution times, and which are sufficiently efficacious at prolonged periods, foamer conditions are set as follows; pump speed about 25-30 rpm, air flow rate about 100-250 (cc)ml/min (at 100 psig input pressure), and rotor speed about 1000 - 2300 rpm. The foam that results generates a back pressure of about 10-40 psig during extrusion. Of course, the conditions are approximate since operational variability occurs in the meters during operation of the foamer. In addition, the dispersion probably has some air in it due to the dispersion formulation step. Prior degassing will likely alter the density of the solution and require a change in the liquid/air ratio in the foamer.

Any of these process variables can be changed, thereby changing the liquid density of the foam. In order to determine how the liquid density of the foam as well as the characteristics of the wound facing layer produced from that foam are affected, foams are manufactured using the foamer wherein one process variable is varied while all other parameters remain constant.

For example, a series of foams of varying liquid density can be produced by varying the flow rate of the incoming gas. The liquid density of the resulting foam is determined using techniques that are well-known in the art. A curve is subsequently generated by plotting the density of the foamed liquid dispersion versus the flow rate of the incoming gas. The foamed dispersions from each run is then lyophilized (that is, freeze-dried under vacuum). Upon lyophilization, the wound facing layer (whose foamed liquid density is known) is evaluated to determine whether it has certain characteristics, including an adequate dissolution time. In this manner it is possible to determine the liquid density which, upon lyophilization, results in a dried foam having a combination of suitable properties. Thus, the settings necessary to foam the dispersion to the desired liquid density can be readily determined empirically or from the graph, and more importantly, controlled by the operator of the mixer who can easily reset the gas flow rate or any other process variable to the appropriate settings.

Therefore, by using the method of the invention one can substantially control the liquid density of the foamed dispersions, and ultimately, the properties, including dosage, dosage delivery rates and dissolution times of the wound facing layer so produced.

Foaming can be continued until the back pressure gauge reaches an equilibrium value. Alternatively, one skilled in the art can readily determine when sufficient foaming has occurred by inspecting the viscosity of the foamed dispersion as it is extruded. Preferably, the density of the liquid foamed dispersion should range from about 0.1 to about 1.0 gm/(cc)ml to afford a suitable dry foam. Liquid foam densities of about 0.4 - 0.6 gm/(cc)ml are more preferred. It has been found that it is important to closely monitor the density of the liquid foams in order to achieve uniformity in dosage and active ingredient delivery rates.

In the next step, the foamed liquid dispersion is placed into a receptacle having a known volume ("unit dosage"). Since the liquid density of the foam and the volume of the receptacle are known, it is a simple calculation to determine the foam weight and the amount of active ingredient incorporated into each unit dosage.

Accordingly, one skilled in the art can readily manufacture batches of dressings containing known and substantially equivalent dosages of active ingredient.

Although the liquid foam can be cast into sheet form, it is preferably extruded through tygon tubing into a preformed mold. Various aluminum, plastic and release liner covered molds can be employed. Polyethylene molds are preferred, since the foams easily release from these molds without cohesive failure.

It is also preferred to extrude the foam into compartmentalized trays whereby the volume of one compartmental unit equals the volume of the resulting wound facing layer. This prevents uncontrolled spread or flow of the foam and thus, the manufacture of dressings which have nonuniform dimensions and dosages.

The foamed dispersion is then lyophilized in a freeze drier in order to generate open cell foams which contain active ingredient. A Virtis 800L-Freezemobile 12 is preferred. The freeze-drier shelves are chilled to below about -40°C. The condenser is chilled to below about -50°C. The filled molds are placed on the shelves and frozen to shelf temperature. The frozen foam is then exposed to the full vacuum (10 - 90 millitorrs) of the unit. Once this vacuum is achieved, the shelf temperature is gradually increased to about room temperature and sublimation continues, preferably for at least about 15 hours, or until the sample temperature reaches about 20 - 25 °C.

Thermal gravimetric analysis may be used to determine the water content of the foams. It may also be used to determine the thermal stability of the wound facing layers by determining degradative weight loss. Typically, residual water is present in amounts of from about 1% to 10% by weight of the final dried product.

The active ingredient content of the foams may be determined using ultraviolet, high pressure liquid chromatography ("HPLC"), or any other known analysis of a solution of the dissolved foam or analysis of the dried bioerodible foams.

The foam product can be cut into the desired size, shape or thickness and applied directly to the site to be treated. Alternatively, the lyophilized foams can be utilized in combination with well-known absorbent or nonabsorbent dressings, including occlusive dressings, and/or adhesive means useful for securing the lyophilized foam product to the area to be treated. Examples of such products include gauze, bandages and the like. Means for combining the foams of the invention with other dressings and/or other adhering means are well-known to those of ordinary skill in the art. For example, well-known laminating or extruding techniques may be used.

Upon contact with the wound, exudate is absorbed by the wound facing layer and the wound facing layer begins to dissolve. As indicated above, the foams of the invention are highly absorbent and can absorb about 3 to 6 times of their own weight of exudate, and even more of water. This property is critical to the effectiveness of the wound dressing since it is the basis for its absorbent capacity, non-reinjury characteristics and active ingredient release tendencies. It is noted that absorption of fluid by a less dense foam is faster than absorption by a more dense foam. For example, it has been determined that a foam having a liquid density of 1.0 gm/(cc)ml and a dry density of 0.02 gm/(cc)ml absorbs about 3.5 gm/gm in the first hour, whereas a foam having a liquid density of 0.5 gm/(cc)ml and a dry density of 0.01 gm/cc absorbs about 5.3 gm/gm in the first hour.

As the exudate is being absorbed, the wound facing layers slowly start to dissolve and release active ingredient. Using the methods of the invention, active ingredients may be continually released at a rate sufficient to maintain a therapeutic or effective level of active ingredient for at least 2 hours, more preferably for at least 4 hours, even more preferably for at least 8 hours, most preferably for up to at least 20 hours.

Upon dissolution, the lyophilized foams first form a gel (i.e., a colloidal solution having the consistency of jelly), and then further dissolve into a liquid. The gels so formed possess good structural integrity for a prolonged period prior to further dissolution to a liquid.

For example, at least 2 hours after the wound facing layers of the invention have been placed in aqueous solution at 37 °C, a substantial amount of gel (at least about 10% by volume) still remains visible to the naked eye. This feature facilitates healing of the wound, permits the dressing to be worn comfortably and helps to control the rate of delivery of the active ingredient.

The time required for the foam-containing-wound facing layers of the invention to attain substantially complete in-vitro dissolution to a liquid (i.e., no gel is evident), as measured by the method described below, is referred to as "dissolution time". By applying the teachings of this invention, dissolution times of at least about 2 hours can be obtained, preferably at least about 4 hours, more preferably at least about 8 hours, even more preferably at least about 20 hours, and most preferably at least about 24 hours. Moreover, in-vivo dissolution times are likely be even greater.

The procedure described in USP XXII, 711 DISSOLUTION, Apparatus 1, from U.S. Pharmacopeia, was followed to determine the dissolution time. This procedure uses an assembly consisting of a covered glass vessel (a Bell jar), a motor, a drive shaft, a basket, and a constant temperature water bath. The speed regulating device used allows the shaft rotation to be selected and maintained at a rate of 35 rpm. The basket is affixed to the drive shaft. The vessel is filled with 400 ml of 1% (by weight) saline solution (pH 5-6). The device is placed into a dry basket at the beginning of each test, and the basket is immersed in the vessel containing the saline solution. The vessel is then immersed in a constant temperature water bath set at 37°C. The sample is observed, and as dissolution takes place, the time for total gelation is recorded. The test is allowed to continue and the time for total dissolution of the gel is also recorded.

In addition, the wound facing layers of the invention are flexible and soft. As mentioned above, it has been determined that the flexibility of the foams is due in part to the liquid density of the foams. For example, a foam having a liquid density of 1.0 gm/(cc)ml and a dry density of 0.02 gm/cc required 1.29 gm of mass to bend it a measured amount. By contrast, lyophilized foams of the invention having a liquid density of 0.5 gm/(cc)ml and a dry density of 0.01 gm/(cc)ml required only 0.28 gm of mass to bend it to the same degree. Flexibility was measured using a Gurley Stiffness Tester, manufactured by W. and L.E. Gurley of Troy, New Jersey. The tester measures the stiffness of a material by defining the resistance offered by that material to bending when an external force is applied. The test is performed in accordance with Tappi 7543 pm-84.

The invention is further illustrated by the following examples which are not intended to be limiting.

### EXAMPLES

### EXAMPLE 1 - Preparation Of A wound Dressing

**1. Dispersion Preparation**
   Deionized water (750 g) was preheated to 95 °C, and 20 g of hydroxypropylmethylcellulose (HPMC) (Methocel E4M-Dow Chemical, Midland, Michigan) was added with stirring. The solution was cooled to 4°C and stirred until all particles were completely wetted and a clear solution resulted. Then, 250 g of deionized water and 10 mg of EGF from Chiron of Emeryville, California (as a 1 mg/ml stock solution) were added and stirred until the EGF was dissolved. EGF is recombinant Human Epidermal Growth Factor.
**2. Lyophilization Procedure**
   The resulting solution was then spread on a tray at a depth of about 8 mm. The tray placed on the shelf of a Virtis freeze drier (Unitop 800L with Freezemobile 12). These shelves were pre-chilled to -40°C. The solution was allowed to freeze to shelf temperature. The frozen solution was lyophilized at full vacuum (20-60 millitorr) to a terminal temperature of 22°C. The drying time was about 24 hours.
   The resulting foam was a white sponge-like material.

### EXAMPLE 2 - Kinetics of Release of Active Ingredient

Another foam was prepared using the ingredients and method set forth in Example 1, except that 10 mg of EGF was replaced with the same amount of radiolabeled EGF. A section of this foam was cut and placed on a silkscreen membrane in a Franz diffusion cell. The receiving reservoir was filled with phosphate buffered saline/bovine serum albumin and maintained at 37°C. The material was 33 millimolar with respect to phosphate; had a saline concentration of 0.9 wt%; and a bovine serum albumin content of 1 mg/mL. Aliquots were removed at specific times and analyzed for the presence of radiotracer using a gamma counter. An equal volume of buffer was added to the diffusion cell to replace each aliquot.

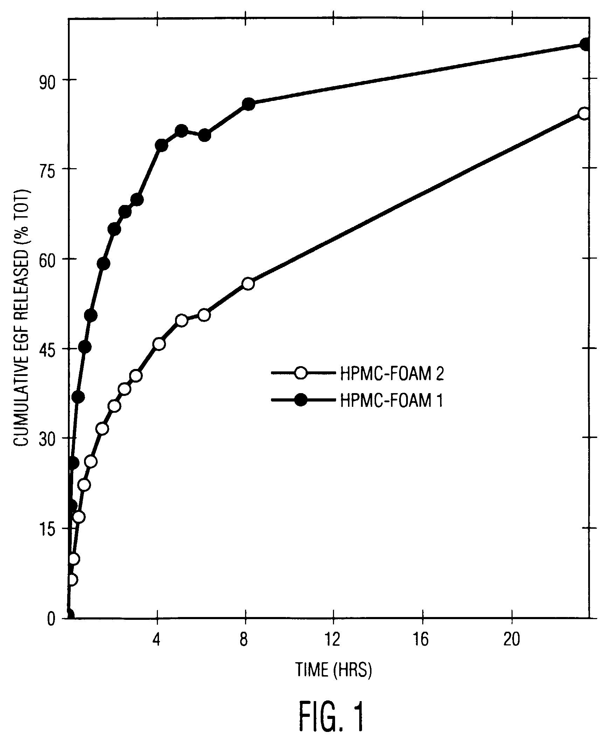

The radioactivity data from each aliquot was used to calculate the percentage of EGF released. The results were plotted as a function of time and are reported in Figure 1.

### EXAMPLE 3 - Preparation Of A wound Dressing

**1. Dispersion Preparation**
   A solution containing 2% gelatin was prepared as in Example 1. The solution was allowed to cool to room temperature.
**2. Foaming Procedure**
   The solution of step 1 was added to the hopper of an Oakes foamer (Model 2MT. 5A). The rotor of the mixing head assembly was set at 1100 rpm and turned on. The flow rate was 350 (cc)ml/min. A white spreadable foam resulted from this procedure. The foam was spread on stainless steel sheets at a depth of 1 cm. The top of the tray was scraped to eliminate excess foam and to produce a smooth, even surface.
**3. Lyophilization Procedure**
   The same procedure described in Example 1 was repeated, except that the frozen solution was lyophilized at full vacuum (20-60 millitorr) to a terminal temperature of 15°C. Drying time was about 48 hours.

The resulting foam was a dry, sponge-like material. The foams readily absorbed water and quickly gelled. The gels dissolved slowly over several hours.

### EXAMPLE 4 - Preparation Of A wound Dressing Containing A Mixture Of Polymers

Any polymers that are compatible in solution and with the active ingredient to be used in the dressing may be blended to modify the properties of the resulting dry foam. In this example, methylcellulose is blended with gelatin to retard the rate of water absorption and dissolution of the foam.
**1. Dispersion Preparation**
   Deionized water (480 g) was preheated to 95 °C, and 20 g of methylcellulose (MC) (from Dow Chemical) was added with stirring. The solution was cooled to 4°C and stirred until all particles were completely wetted and a clear solution resulted. The clear solution is set aside.
   Deionized water (470 g) was preheated to 90 °C, and 30 g of gelatin was added with stirring. The solution is stirred until a uniform solution resulted. The gelatin and MC solutions are allowed to equilibrate to room temperature (about 20 °C).
   Then, the two solutions are combined, first by mixing by hand with a spatula, and then by mixing at low speed with a power mixer. A uniform solution is obtained.
**2. Foaming Procedure**
   The solution of step 1 was added to the hopper of an Oakes Foamer (Model 2MT. 5A) and the solution was treated as in Example 3. A white spreadable foam resulted. The foam was spread on stainless steel sheets at a depth of 1 cm.
**3. Lyophilization Procedure**
   The procedure described in Example 3 was repeated.

The resulting foam was a dry, sponge-like material that was softer than the foam produced in Example 3. The foams absorbed water more slowly and required more time to completely dissolve than those prepared in accordance with Example 3.

### EXAMPLE 5 - Preparation Of A Wound Dressing Containing

### Mannitol

In this example, mannitol is blended with HPMC to increase the rate of water absorption and dissolution of the foam.
**1. Dispersion Preparation**
   Deionized water (750 g) was preheated to 95 °C, and 4 g of mannitol is dissolved in the water. Then, 20 g of HPMC was added with stirring. The solution was cooled to 4°C and stirred until all particles were completely wetted and a clear solution resulted.
**2. Foaming Procedure**
   The resulting dispersion was foamed using the
   conditions described in Example 3. The foam was spread on a stainless steel sheet lined with wax paper at a depth of about 7 mm.
**3. Lyophilization Procedure**
   The procedure described in Example 3 was repeated, except that the dispersion was prechilled to -40°C and lyophilized to 22°C for 24 hours.

Another foam was prepared which was identical to the one herein described except that it did not contain mannitol.

The resulting mannitol-containing foam absorbed water more quickly than those which did not contain mannitol. In addition, the mannitol-containing foams required less time to completely dissolve.

### EXAMPLE 6 - Preparation Of A Wound Dressing Containing Ketanserin Tartrate

**1. Dispersion Preparation**
   Deionised water (660 g) was preheated to 95 °C, and 40 g of HPMC (Methocel E4M-Dow Chemical, Midland, Michigan) was added with stirring. The solution was stirred until all particles were completely wetted. Then, 1300 g of deionized water was added at 15 to 20°C. This solution was stirred for about 20 minutes. The solution was then allowed to degas on standing. A clear solution resulted.
   Then about 13 g of ketanserin tartrate (2% solution) supplied by Janssen Pharmaceutica was added to about 1985 g of the HPMC solution and stirred until the ketanserin tartrate was dissolved.
**2. Foaming Procedure**
   The solution of step 1 was added to the hopper of an Oakes Foamer (Model 2MT. 5A). The solution was pumped through the system at a speed of about 25 rpm until equilibrium was attained. The initial 100 - 150 ml was purged and discarded. The remainder was recirculated. The rotor of the mixing head assembly was set at 1500 rpm and turned on. The air pressure was set at 100 psig and a flow rate of 220 (cc)ml/min. Pump speed was maintained at about 26.5 rpm. The foam was generated at a back pressure of about 40 to 45 psi.
   The foam was then exuded through 1/4" tygon tubing into compartmentalized trays having single cell dimensions of 8 x 10 x 0.8 cm. The top of the tray was scraped to eliminate excess foam and to produce a smooth, even surface.
**3. Lyophilization Procedure**
   The tray placed on the shelf of a Virtis freeze drier (Unitop 800L with Freezemobile 12). These shelves were pre-chilled to -45°C. The solution was allowed to freeze to shelf temperature. The frozen solution was lyophilized at full vacuum (20-60 millitorr) to a terminal temperature of 20°C. The drying time was about 16 hours.

### Drug Assay

The quantity of ketanserin tartrate in the dry foam was measured by dissolution of the foams and HPLC analysis of the solution. Assay of the ketanserin tartrate-containing foams was achieved by dissolving the foams in 500 ml of distilled water with stirring for 24 hours. Then 0.5 ml of this solution was diluted to 10 ml with distilled water. This sample was then analyzed using a Waters HPLC. The HPLC conditions were as follows:
- Column: Hypersil (Keystone Scientific)
ODS 250 x 4.6mm
- Mobile Phase: Ammonium acetate in H₂O (0.5 w/v):
Methanol (0.25-0.75)
- Flow rate: 1.2 ml/min
- Wavelength: 240 nm
- Retention Time: 6 mins

In an analysis of 10 samples, the average drug dose was 187.75 mg +/- 13.22 mg. The uniformity of dosage among samples was reflected in a small coefficient of variation of about 7.0%.

### In-Vitro Foam Dissolution Studies

The dissolution times of high and low density foams and foams containing active ingredient were examined. Samples of foam (8 x 10 x 0.8 cm) were laid on the surface of 100 ml of 1% saline solution at room temperature without stirring. The dissolution times were recorded based on visual inspection.

Table I presents data on the dissolution times achieved by foams representative of those used in the invention (Examples 4-9) and comparative examples (Examples 1-3). In all cases the foams of the invention required more than 24 hours to completely dissolve, whereas the foams of the prior art dissolved in a much shorter time. In addition, with absorption of fluid the foams of the invention slowly formed a gel which slowly fragmented into discreet gel particles. It was further observed that the higher density foams of the invention required more time to dissolve and the resulting gels had greater cohesive strength.

**TABLE I**

| **Sample** | **Dissolution Time-hrs********* |
|---|---|
| 1. (6% PVA solution)***** | 7.5 |
| 2. (6% PVA foam)***** | 4 |
| 3. (3:1:1 gelatin/pectin/CMC)****** | 4.75 |
| 4. High density placebo (1.59 g) | > 24 hours |
| 5. High density placebo (1.65 g) | > 24 hours |
| 6. Low density placebo (0.83 g) | > 24 hour: |
| 7. Low density placebo (0.80 g) | > 24 hours |
| 8. drug containing foam (0.99 g) | > 24 hours |
| 9. drug containing foam (0.98 g) | > 24 hours |

| | |
|---|---|
| ***** The percentage indicates the percentage of polymer added to the liquid dispersion by weight (prior to foaming). | |
| ****** The ratio indicates the initial ratio of polymers added to the aqueous dispersion. | |
| ******* Sample 1-3 determined by previously described USP method. Sample 4-9 determined by previously described surface technique. | |

In comparative examples 1 and 2, the foam was comprised of polyvinylalcohol (Airvol 540, Air Products, Allentown, Pennsylvania) and was prepared in accordance with the teachings of Japanese Patent Application No. 78-145170. The foam of example 3 was prepared in accordance with the description set forth in U.S. patent No. 4,292,972, Example 3.

Samples 4 - 9 were comprised of foams containing hydroxypropylmethylcellulose (Methocel E4M, obtained from the Dow Chemical Company, Midland, Michigan). Samples 4 and 5 were placebos containing no active ingredient and had a high liquid density of 1 g/(cc)ml. These samples had a dry density of 0.029/(cc)ml. Samples 6 and 7 were also placebos but of a low density of 0.5 g/(cc)ml. These samples had a dry density of 0.01 g/(cc)ml. Samples 8 and 9 contained ketanserin tartrate and were made in accordance with Example 6.

As the data above shows, the foams of the prior art dissolved in about 4 to 8 hours. These foams exhibited poor structural integrity. The foam produced in accordance with the teachings of the Japanese Patent Application was too stiff.

On the other hand, the foams of the invention were soft and pliable, exhibited good structural integrity over time, and had good absorption. Moreover, as indicated above, they had dissolution times of over 24 hours. These physical properties result in a wound dressing that is comfortable, and which provides a proper healing environment for chronic wounds.

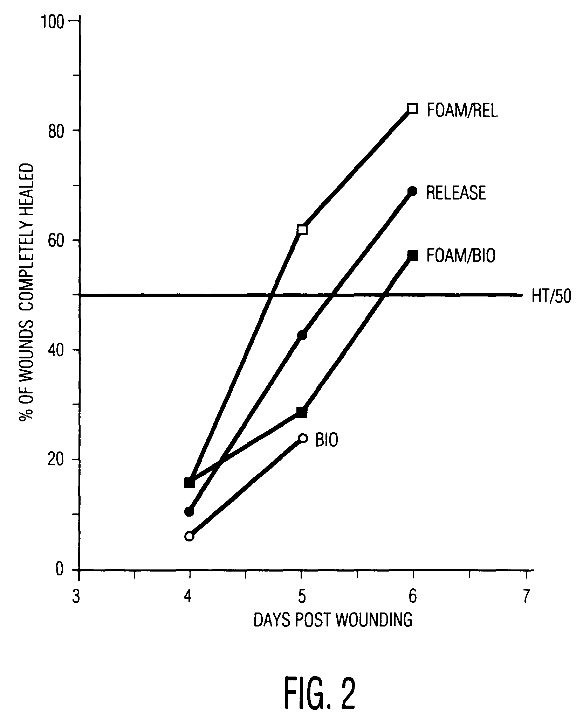

The wound care attributes of the wound dressings without active ingredient were observed in a partial thickness wound model using Yorkshire pigs. In this model, 32 wounds 8 x 15 x .5 mm were made with a dermatome on the right and left thoracic region of the pig. The wounds were treated with the foam samples under an occlusive dressing (Bioclusive Transparent Film Dressing sold by Johnson & Johnson, New Brunswick, NJ) and a non-occlusive secondary gauze dressing (Release™) taped in place with Dermacel™ Tape. Healing rates for the occlusive dressing were acceptable. The hydroxypropylmethylcellulose foam absorbed exudate readily and did not reinjure upon removal. The healing rate of the foam under occlusion and non-occlusion relative to the occlusive control is shown in Figure 2.

The invention having now been fully described, it should be understood that it may be embodied in other specific forms or variations without departing from its spirit or essential characteristics. Accordingly, the embodiments described above are to be considered in all respects as illustrative and not restrictive, the scope of the invention being indicated by the appended claims rather than by the foregoing description, and all changes which come within the meaning and range of equivalency of the claims are intended to be embraced therein.

## Claims

1. A wound facing layer comprising a water-soluble lyophilized foam comprising at least one water-soluble polymer and having a density of 0.01 to 0.1 g/ml(cc), an absorbent capacity of 300% to 600% and a dissolution time of at least 2 hours.

2. The layer of Claim 1, wherein said water-soluble polymer is at least one of polyurethane, chitsonium salt, sodium alginate, polyvinyl alcohol, methylcellulose, sodium carboxymethylcellulose, hydroxyethylcellulose, hydroxypropylcellulose, hydroxyethylmethylcellulose, hydroxyethylethylcellulose, hydroxypropylethylcellulose, hydroxypropylmethylcellulose, gelatin, polyvinylpyrrolidone, or a mixture thereof.

3. The layer of Claim 2, wherein said foam comprises hydroxypropylmethylcellulose.

4. The layer of any one of Claims 1 to 3 wherein said foam additionally comprises a preservative.

5. The layer of any one of Claims 1 to 4 which has a dissolution time of at least 4 hours, preferably at least 8 hours and most preferably at least 20 hours.

6. The layer of any one of Claims 1 to 5, which dissolves into a gel prior to dissolution to a liquid.

7. The layer of any one of Claims 1 to 6, wherein the foam has a density of 0.01 to 0.05 g/(cc)ml, preferably 0.01 to

8. The layer of any one of Claims 1 to 7, further comprising an active ingredient, such as growth factors, hormones, anti-inflammatories, analgesics, sertonin antagonists, and anti-microbials.

9. The layer of Claim 8, wherein said active ingredient comprises up to 95%, preferably from 20% to 70%, by weight of said layer.

10. The layer of Claim 8 or Claim 9, which is a substantially homogeneous mixture of foam and active ingredient.

11. The layer of any one of Claims 8 to 10, wherein the active ingredient is continually releasable over a period of at least 2 hours.

12. The layer of any one Claims 8 to 11 for use in the controlled delivery of an active ingredient to a wound, such as a decubitus ulcer, a venous stasis ulcer, a diabetic ulcer, a cutaneous ulcer, a burn, a donor site, psoriasis or an incision.

13. A method for manufacturing a wound facing layer comprising a water-soluble lyophilised foam, said method comprising:
(a) forming an aqueous dispersion comprising at least one water-soluble polymer;
(b) providing a continuous, enclosed mixer capable of operating under pressure to foam the dispersion, said continuous mixer comprising: a pump; a mixing chamber; a mixer assembly having a rotor, a gas inlet and an outlet for the foamed dispersion; means to measure a pump speed, a rotor speed, a flow rate and pressure of an incoming gas; and means to measure a back pressure of the foamed dispersion;
(c) selecting a target density for the foamed liquid dispersion;
(d) setting a pump speed;
(e) setting a rotor speed;
(f) setting a pressure and flow rate for an incoming gas;
(g) increasing the viscosity of said dispersion of step (a) until it has a viscosity sufficient to foam;
(h) transferring said dispersion to said mixer, and then foaming said dispersion until the density of the dispersion is substantially equal to the target density;
(i) measuring the back pressure of the foamed dispersion;
(j) placing said foamed dispersion into a receptacle of known volume; and
(k) lyophilizing said dispersion to produce a lyophilized foam.

14. The method of Claim 13 wherein: step (a) comprises forming a dispersion further comprising an active ingredient; and step (c) further comprises selecting a target dosage of active ingredient for said layer, whereby after step (k) the dosage of active ingredient is substantially equal to the target dosage.

15. The method of Claim 14 wherein step (j) comprises extruding said dispersion into a compartmentalized tray comprised of identical units of known volume, whereby the dosage of active ingredient in each unit is substantially equal to the target dosage.

16. The method of any one of Claims 13 to 15 wherein step (a) comprises providing polymer in the dispersion at 0.5% to 10% by weight, preferably 1% to 4% by weight, most preferably about 2% by weight.

17. The method of any one of Claims 13 to 16 wherein the liquid density of the dispersion of step (h) is 0.1 to 1.0 g/(cc)ml preferably 0.4 to 0.6 g/(cc)ml.

18. The method of any one of Claims 13 to 17 wherein said dispersion is lyophilized in a freeze-drier at full vacuum for at least 15 hours.

## Patentansprüche

1. Wundenseitige Schicht enthaltend einen wasserlöslichen, gefriergetrockneten Schaum, der mindestens ein wasserlösliches Polymer enthält und eine Dichte von 0,01 bis 0,1 g/ml, ein Absorptionsvermögen von 300% bis 600% und eine Auflösungszeit von mindestens 2 Stunden aufweist.

2. Schicht nach Anspruch 1, wobei das wasserlösliche Polymer enthält: mindestens ein Polyurethan, Chitsonium-Salz, Natriumalginat, Polyvinylalkohol, Methylcellulose, Natriumcarboxymethylcellulose, Hydroxyethylcellulose, Hydroxypropylcellulose, Hydroxyethylmethylcellulose, Hydroxyethylethylcellulose, Hydroxypropylethylcellulose, Hydroxypropylmethylcellulose, Gelatine, Polyvinylpyrrolidon oder eine Mischung davon.

3. Schicht nach Anspruch 2, wobei der Schaum Hydroxypropylmethylcellulose enthält.

4. Schicht nach irgendeinem der Ansprüche 1 bis 3, wobei der Schaum zusätzlich einen Konservierungsstoff enthält.

5. Schicht nach irgendeinem der Ansprüche 1 bis 4, die eine Auflösungszeit von mindestens 4 Stunden, bevorzugt mindestens 8 Stunden und am meisten bevorzugt mindestens 20 Stunden aufweist.

6. Schicht nach irgendeinem der Ansprüche 1 bis 5, die sich vor der Auflösung in eine Flüssigkeit in ein Gel auflöst.

7. Schicht nach irgendeinem der Ansprüche 1 bis 6, wobei der Schaum eine Dichte von 0,01 bis 0,05 g/ml und bevorzugt 0,01 bis 0,03 g/ml aufweist.

8. Schicht nach irgendeinem der Ansprüche 1 bis 7, zusätzlich enthaltend einen Wirkstoff wie zum Beispiel Wachstumsfaktoren, Hormone, entzündungshemmende Stoffe, Analgetika, Serotonin-Antagonisten und antimikrobielle Stoffe.

9. Schicht nach Anspruch 8, wobei der Wirkstoff bis zu 95 % und bevorzugt zwischen 20% und 70% des Gewichts der Schicht enthält.

10. Schicht nach Anspruch 8 oder Anspruch 9, die substantiell eine homogene Mischung aus Schaum und Wirkstoff ist.

11. Schicht nach irgendeinem der Ansprüche 8 bis 10, wobei der Wirkstoff über einen Zeitraum von mindestens 2 Stunden kontinuierlich freisetzbar ist.

12. Schicht nach irgendeinem der Ansprüche 8 bis 11 für die Anwendung bei der kontrollierten Zuführung eines Wirkstoffs zu einer Wunde, wie zum Beispiel einem Dekubitalgeschwür, einem Venenstauungsgeschwür, einem Diabetesgeschwür, einem Hautgeschwür, einer Verbrennung, einer Spenderstelle, einer Psoriasis-Stelle oder einer Schnittwunde.

13. Verfahren zur Herstellung einer wundenseitigen Schicht enthaltend einen wasserlöslichen, gefriergetrockneten Schaum, wobei das Verfahren folgende Schritte umfaßt:
(a) Bildung einer wäßrigen Dispersion enthaltend mindestens ein wasserlösliches Polymer;
(b) Bereitstellung eines kontinuierlichen, geschlossenen Mischers, der unter Druck betrieben werden kann, um die Dispersion zu schäumen, wobei der kontinuierliche Mischer eine Pumpe; eine Mischkammer; eine Mischeinheit mit einem Rotor, eine Gaseinströmöffnung und eine Ausströmöffnung für die geschäumte Dispersion; eine Vorrichtung zum Messen der Pumpengeschwindigkeit, der Rotorgeschwindigkeit, der Durchströmmenge und des Drucks des einströmenden Gases; und eine Vorrichtung zum Messen des Gegendrucks der geschäumten Dispersion, umfaßt;
(c) Auswahl der beabsichtigten Dichte für die geschäumte Flüssigdispersion;
(d) Einstellung der Pumpengeschwindigkeit;
(e) Einstellung der Rotorgeschwindigkeit;
(f) Einstellung des Drucks und der Durchströmmenge für das einströmende Gas;
(g) Erhöhung der Viskosität der Dispersion von Schritt (a), bis die Dispersion die für die Schaumbildung ausreichende Viskosität aufweist;
(h) Weiterleitung der Dispersion zum Mischer, und anschließend Schäumung der Dispersion, bis die Dichte der Dispersion der beabsichtigten Dichte substantiell gleicht;
(i) Messung des Gegendrucks der geschäumten Dispersion;
(j) Einbringung der geschäumten Dispersion in einen Behälter bekannten Volumens; und
(k) Gefriertrocknung der Dispersion zur Herstellung eines gefriergetrockneten Schaums.

14. Verfahren nach Anspruch 13, wobei: Schritt (a) die Bildung einer Dispersion, die zusätzlich einen Wirkstoff enthält, umfaßt; und Schritt (c) zusätzlich die Auswahl der beabsichtigten Dosierung des Wirkstoffs für die Schicht umfaßt, wodurch nach Schritt (k) die Dosierung des Wirkstoffs der beabsichtigten Dosierung substantiell gleicht.

15. Verfahren nach Anspruch 14, wobei Schritt (j) die Extrusion der Dispersion in eine aufgegliederte Schale bestehend aus identischen Einheiten bekannten Volumens einschließt, wodurch die Dosierung des Wirkstoffs in jeder Einheit substantiell der beabsichtigten Dosierung gleicht.

16. Verfahren nach irgendeinem der Ansprüche 13 bis 15, wobei Schritt (a) den Zusatz eines Polymers in der Dispersion zu 0,5% bis 10% des Gewichts, bevorzugt 1% bis 4% des Gewichts und am meisten bevorzugt ungefähr 2% des Gewichts, umfaßt.

17. Verfahren nach irgendeinem der Ansprüche 13 bis 16, wobei die Flüssigkeitsdichte der Dispersion von Schritt (h) 0,1 bis 1,0 g/ml und bevorzugt 0,4 bis 0,6 g/ml beträgt.

18. Verfahren nach irgendeinem der Ansprüche 13 bis 17, wobei die Dispersion in einem Gefriertrockner bei vollständigem Vakuum für mindestens 15 Stunden gefriergetrocknet wird.

## Revendications

1. Couche pour revêtement de blessure comprenant une mousse lyophilisée soluble dans l'eau comprenant au moins un polymère soluble dans l'eau ayant une densité de 0,01 à 0,1 g/ml, une capacité d'absorption de 300% à 600% et un temps de dissolution d'au moins 2 heures.

2. Couche selon la revendication 1, dans laquelle ledit polymère soluble dans l'eau consiste en au moins un composé parmi le polyuréthanne, un sel de chitosonium, l'alginate de sodium, le poly(alcool vinylique), la méthylcellulose, la carboxyméthylcellulose sodique, l'hydroxyéthylcellulose, l'hydroxypropylcellulose, l'hydroxyéthylméthylcellulose, l'hydroxyéthyléthylcellulose, l'hydroxypropyléthylcellulose, l'hydroxypropylméthyl-cellulose, la gélatine, la polyvinylpyrrolidone, ou un de leurs mélanges.

3. Couche selon la revendication 2, dans laquelle ladite mousse comprend de l'hydroxypropylméthylcellulose.

4. Couche selon l'une quelconque des revendications 1 à 3, dans laquelle ladite mousse comprend en plus un conservateur.

5. Couche selon l'une quelconque des revendications 1 à 4 qui a un temps de dissolution d'au moins 4 heures, de préférence d'au moins 8 heures et le plus préférablement d'au moins 20 heures.

6. Couche selon l'une quelconque des revendications 1 à 5, qui se dissout en un gel avant dissolution en un liquide.

7. Couche selon l'une quelconque des revendications 1 à 6, dans laquelle la mousse a une densité de 0,01 à 0,05 g/ml, de préférence de 0,01 à 0,03 g/ml.

8. Couche selon l'une quelconque des revendications 1 à 7, comprenant en outre un ingrédient actif tel que des facteurs de croissance, des hormones, des anti-inflammatoires, des analgésiques, des antagonistes de la sérotonine, et des anti-microbiens.

9. Couche selon la revendication 8, dans laquelle ledit ingrédient actif constitue jusqu'à 95%, de préférence de 20% à 70%, en poids de ladite couche.

10. Couche selon la revendication 8 ou la revendication 9, qui est un mélange essentiellement homogène de mousse et d'ingrédient actif.

11. Couche selon l'une quelconque des revendications 8 à 10, dans laquelle l'ingrédient actif est libérable continuellement sur une période d'au moins 2 heures.

12. Couche selon l'une quelconque des revendications 8 à 11, destinée à être utilisée dans la délivrance contrôlée d'un ingrédient actif à une blessure, telle qu'un ulcère de décubitus, un ulcère de stase veineuse, un ulcère diabétique, un ulcère cutané, une brûlure, un site donneur, un psoriasis ou une incision.

13. Procédé de préparation d'une couche pour revêtement de blessure comprenant une mousse lyophilisée soluble dans l'eau, ledit procédé comprenant :
(a) la formation d'une dispersion aqueuse comprenant au moins un polymère soluble dans l'eau;
(b) la fourniture d'un mélangeur fermé, continu, capable de fonctionner sous pression pour faire mousser la dispersion, ledit mélangeur continu comprenant : une pompe; une chambre de mélange; un ensemble mélangeur ayant un rotor, une entrée de gaz et une sortie pour la dispersion transformée en mousse; des moyens pour mesurer une vitesse de pompe, une vitesse de rotor, un débit d'écoulement et une pression de gaz entrant; et des moyens pour mesurer une contre-pression de la dispersion transformée en mousse;
(c) la sélection d'une densité-cible pour la dispersion liquide transformée en mousse;
(d) le réglage d'une vitesse de pompe;
(e) le réglage d'une vitesse de rotor;
(f) le réglage d'une pression et d'un débit d'écoulement pour un gaz entrant;
(g) l'augmentation de la viscosité de ladite dispersion de l'étape (a) jusqu'à ce qu'elle ait une viscosité suffisante pour mousser;
(h) le transfert de ladite dispersion dans ledit mélangeur, puis le moussage de ladite dispersion jusqu'à ce que la densité de la dispersion soit sensiblement égale à la densité-cible;
(i) la mesure de la contre-pression de la dispersion transformée en mousse;
(j) la mise en place de ladite dispersion transformée en mousse dans un réceptacle de volume connu; et
(k) la lyophilisation de ladite dispersion pour produire une mousse lyophilisée.

14. Procédé selon la revendication 13, dans lequel : l'étape (a) comprend la formation d'une dispersion comprenant en outre un ingrédient actif; et l'étape (c) comprend en outre la sélection d'une dose-cible d'ingrédient actif pour ladite couche, ce par quoi après l'étape (k) la dose d'ingrédient actif est sensiblement égale à la dose-cible.

15. Procédé selon la revendication 14, dans lequel l'étape (j) comprend l'extrusion de ladite dispersion en un plateau compartimenté composé d'unités identiques de volume connu, ce par quoi la dose d'ingrédient actif dans chaque unité est sensiblement égale à la dose-cible.

16. Procédé selon l'une quelconque des revendications 13 à 15, dans lequel l'étape (a) comprend l'apport de polymère dans la dispersion à raison de 0,5% à 10% en poids, de préférence de 1% à 4% en poids, le plus préférablement d'environ 2% en poids.

17. Procédé selon l'une quelconque des revendications 13 à 16, dans lequel la densité à l'état liquide de la dispersion de l'étape (h) est de 0,1 à 1,0 g/ml, de préférence de 0,4 à 0,6 g/ml.

18. Procédé selon l'une quelconque des revendications 13 à 17, dans lequel ladite dispersion est lyophilisée dans un appareil de lyophilisation sous vide total pendant au moins 15 heures.
